Europäisches Patentamt

(19) European Patent Office

Office européen des brevets

(11) Publication number: **0 375 706 B1**

# EUROPEAN PATENT SPECIFICATION

(45) Date of publication of patent specification:
26.06.91 Bulletin 91/26

(51) Int. Cl.$^5$: **G07C 9/00, A61B 5/103**

(21) Application number: 88907059.5

(22) Date of filing: 28.07.88

(86) International application number:
PCT/EP88/00685

(87) International publication number:
WO 89/01674 23.02.89 Gazette 89/05

(54) FINGERPRINT DETECTING TERMINAL COMPRISING AN ERGONOMICALLY OPTIMIZED FINGER LOCATION PLATE.

(30) Priority: 11.08.87 EP 87307084

(43) Date of publication of application:
04.07.90 Bulletin 90/27

(45) Publication of the grant of the patent:
26.06.91 Bulletin 91/26

(84) Designated Contracting States:
CH DE FR GB IT LI

(56) References cited:
EP-A- 0 081 276
WO-A-86/00009
GB-A- 1 150 511
US-A- 1 719 950
US-A- 2 384 018

(56) References cited:
US-A- 2 746 192
US-A- 3 516 059
US-A- 4 246 568
US-A- 4 525 859

(73) Proprietor: Siemens Aktiengesellschaft
Wittelsbacherplatz 2
W-8000 München 2 (DE)
Proprietor: Siemens Ltd.
Siemens House Windmill Road
Sunbury-on-Thames Middlesex TW16 7HS
(GB)

(72) Inventor: TIMMS, Ian
22 Padgbury Lane
Congleton Cheshire CW12 4LP (GB)
Inventor: ELLISON, Ian
Klobenstreiner Strasse 2
W-8000 München 90 (DE)

## Description

Fingerprint detecting terminal comprising an ergonomically optimized finger location plate

The invention relates to a fingerprint detecting terminal including a fingerprint sensor with a contact surface for a finger under investigation.

Fingerprint detecting terminals of the type indicated above, having many different shapes and serving different purposes, are previously known.

EP-A-81276 discloses a fingerprint detecting terminal comprising a fingerprint sensor with a contact surface for a finger under investigation and ergonomically optimised finger location plate comprising a primary finger trough for controlling axial location, transverse motion and angular twist of said finger, whereby said contact surface is arranged within said finger location plate and wherein said finger trough is designed ergonomically to suit the relaxed hand shape and position.

European patent application, serial no. 86 10 62 31.3, filed May 1986, incorporated by reference herein, describes an apparatus and procedure for determining the authorization of individuals by verifying their fingerprints. The apparatus of the invention uses a portable data carrier e.g. a plastic card, which has magnetic tracks to store data. Stored data can consist of the degree of correlation between a fingerprint of an authorized individual and a selected reference image, and the code number of the selected reference image. A fingerprint detection terminal with a sensor includes a memory in which the selected reference image is stored. The terminal compares the actual fingerprint of an individual to be checked with the corresponding reference image identified on the plastic card and stored in the fingerprint detection terminal. The determined degree of correlation is in turn compared to the degree of correlation stored on the plastic card. As a function of the result of this comparison, an appropriate decision signal is generated. The data are written on the plastic card in such a way that, based on the data alone, only ambiguous conclusions concerning the identity of the individual are possible.

The apparatus of the invention permits the use of independent, convenient terminals. These terminals can serve to control the entry to and exit from access-controlled security areas. Furthermore, it is possible, for example, to combine a device of this kind with each of the automated teller machines commonly located at banks. Such terminals can also be used to ensure the security of financial transactions of any kind. Thereby, the unlawful use of credit cards, for example, can be almost entirely eliminated.

In order to perform under these conditions, the terminals must be robust and of simple, solid construction. Furthermore, the terminals should be hygienic and comfortable, and the user should not be afraid that he will damage his finger. A wide variety of finger sizes, shapes, and softnesses, and various fingernail lengths will be expected.

It is therefore an object of the invention to provide an ergonomically optimized fingerprint detecting terminal which reliably locates or positions an individual's finger.

The above object is achieved by a fingerprint detecting terminal according to the invention as defined herein.

The previous problems of finger location in a fingerprint detecting terminal are eliminated, because the area of the fingerprint containing the greatest concentration of details or minutiae is reliably located on said contact surface. It has been shown that this detail area may be defined from the nominal line of the distal joint. Consequently, the finger locator ideally also references from this joint. Transverse location of said fingerprint is achieved by providing two guiding rails, arranged in parallel on said contact surface. The guiding rails may be small rectangular bars bounding or limiting said contact surface.

Additionally, in order to control transverse motion and angular twist of the whole finger, to provide a standardized repeatable location, and to obtain a uniform pressure distribution over the entire contact surface, said contact surface is arranged within a finger trough.

This finger trough is designed to suit the shape and position of the relaxed hand, so as to make use more acceptable and comfortable, and to avoid a cramped position of the finger under investigation. Therefore these finger troughs are slightly bowed according to a relaxed hand position.

To improve these comfort features in a preferred embodiment of the invention, one central finger trough comprising said contact surface is arranged with adjacent finger location troughs designed to receive neighbouring fingers of the finger under investigation.

The arrangement and length of adjacent troughs persuades the indiviual to place the appropiate finger under investigation into the middle trough.

This is achieved independently of the individual being a right-or a left-hander.

It has been determined that it is most suitable to arrange the adjacent finger troughs at an angle with respect to each other, within the range of 2-5 degrees.

It is advantageous to incorporate the finger troughs within a housing cover of the terminal, in order to provide a bearing surface area for the hand.

The housing cover can be cleaned easily.

In another embodiment of the invention, guiding elements of a card reader are formed at the adjacent front areas of the housing cover designed as a finger location plate, and a housing enclosing electronic equipment of said terminal.

The invention will be described in the following with reference to embodiments which are shown in the drawings in which :

Fig. 1 is a side view of a fingerprint detecting terminal according to the invention, for use in an access-control system,

Fig. 2 is a top view of the fingerprint detecting terminal of Fig. 1,

Figs. 3a-g are schematic cross-sections through different embodiments of distal joint location ridges,

Figs. 4a-d are schematic cross-sections through different embodiments of means provided for sideways location of a finger under investigation,

Fig. 5 is a perspective view of an embodiment of a finger location plate comprising the locating elements of Figs. 3 and 4, and

Fig. 6 is a schematic cross-sectional view of one embodiment of a finger location plate.

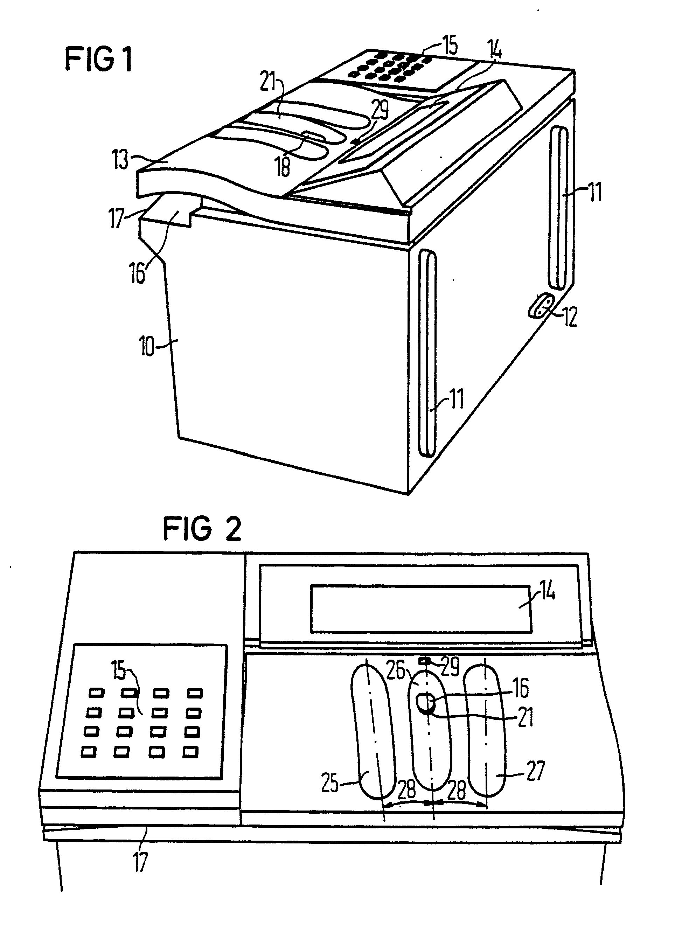

The fingerprint detecting terminal shown in Figs. 1 and 2 comprises a plastic moulded housing 10 in the form of a box which encloses the entire evaluation logic and power supply, and carries mounting elements 11 and power plug 12. The terminal is removably covered by a finger location plate 13, an indication display 14 and an input keyboard 15.

A card reader 16 designed to receive a card-like data carrier is located between the finger location plate 13 and the housing 10, whereby adjacent elements of the finger location plate 13 and the housing 10 form guiding elements 17 of the card reader 16.

A fingerprint sensor 30 with a contact surface 18 is incorporated in the finger location plate 13 and is arranged inside the housing 10. Normally, a contact surface 18 is a pressure sensitive contact surface made of a coated elastomer, for receiving the contact pressure of a finger 19 under investigation. An electric output signal, corresponding to the ridges and valleys of a finger 19 placed on the contact surface 18, is generated by the sensor 30.

The area of the finger 19 containing the greatest concentration of details or minutiae e.g. ridges and valleys, is the finger tip of the index finger or any other finger of either the right or the left hand. This area must be accurately placed on the contact surface 18 in order to ensure that reliable output signals are generated by the fingerprint sensor 30.

Accurate placement will be achieved when a given individual's finger is located so that the area of interest falls on the contact surface, + / – 1 mm, both longitudinally and transversely. It is therefore necessary to provide means for controlling the axial and transverse location of this area of interest.

It has been found that ideal results are achieved when the area of interest is defined from the nominal line of the distal joint 20 of a finger 19. Consequently, the locating or positioning of a finger 19 ideally is also referenced from this joint.

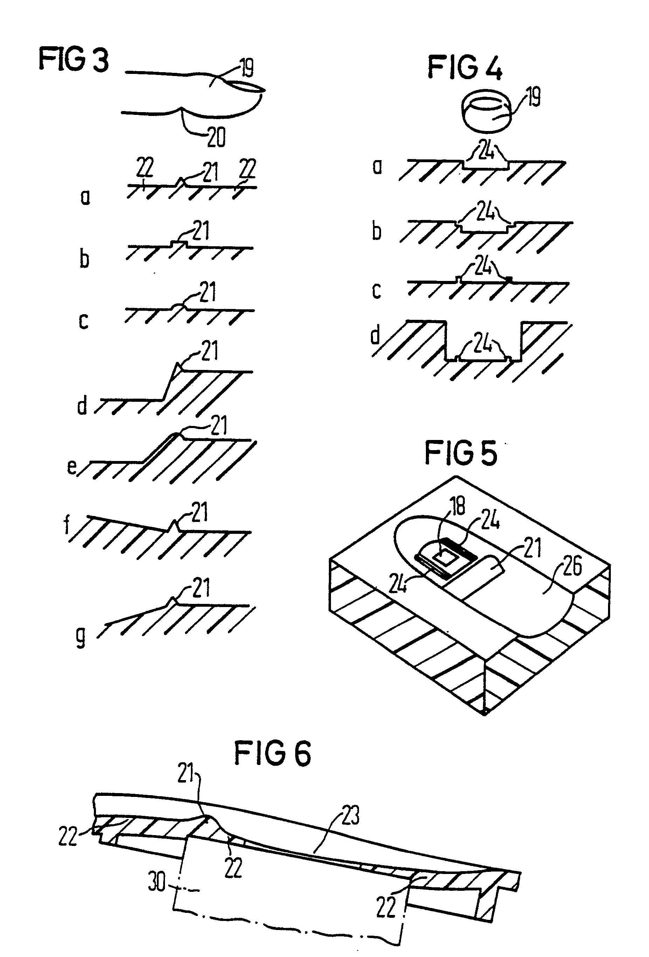

As shown in Fig. 3 as well as in Figs. 1 and 2, a distal joint location ridge 21 is arranged in a finger trough 26, for longitudinally locating a finger 19. The ridge 21 is designed to fit the distal joint 20 of a finger 19, so that it can easily be sensed by a person under investigation.

Several embodiments of location ridges having a wedge- or riblike design are shown in Figs. 3a-g. Finger supporting areas 22 of different slope angles are arranged on both sides of the ridges 21. It has been found that the best results are achieved when the ridge 21 is designed according to the distal joint shape of a finger in a relaxed position. A typical ridge has a height of about 1-3 mm with respect to the contact surface 18 and extends along the entire width of the contact surface 18. However, it has been determined that even different sizes and shapes of ridges may be utilized, provided the person under investigation can feel the ridge and consequently controls his finger location.

As shown in the cross-sectional view of Fig. 6, the location ridge 21 and the adjacent supporting area 22 are designed as a one-piece element made of mouldable plastic material comprising an opening 23 for the contact surface 18. The one-piece element provides side covering for the contact surface 18, whereby the actual opening 23 for the contact surface is about 10 by 10 mm in size, or of a slightly rectangular or oval shape with similar dimensions.

In a further embodiment of the invention shown in Figs. 4 and 5, two guiding rails 24 in the form of small rectangular bars or even edges or grooves are arranged in parallel in order to provide sideways or transverse location of the fingerprint details or minutiae. These guiding rails 24 extend longitudinally along the contact surface 18, preferably bounding or limiting the contact surface. When a person under investigation feels these rails 24, he may adjust his finger tip position according to the direction and position of the rails 24, Thus, the proper sideways location of the fingerprint minutiae on the contact surface 18 is achieved.

Irrespective of control means like ridges or rails associated with the contact surface 18, it has been found most advantageous to provide three finger troughs 25, 26, 27 as shown in Figs. 1 and 2, for controlling transverse motion and angular twist of a finger or even a whole hand. A central finger trough 26 comprising a contact surface 18 near its fingertip end area is designed to receive an index finger, for example. Adjacent finger troughs 25, 27 are designed to receive neighbouring fingers of the finger under investigation. For example, the index finger of the right hand would be placed in trough 26, while the middle finger of the right hand would be placed in trough 27. For the left hand, the index finger would be placed in trough 26 and the middle finger would be placed in trough 25.

To suit a relaxed hand position, the finger troughs 25, 27 are arranged at an angle 28 of about 2 to 5 degrees with respect to the central trough 26, and all of the troughs 25, 26, 27 are slightly bowed or curved in shape.

These finger troughs 25, 26, 27 are an integral part of the finger location plate 13. To give support to the relaxed hand, the one-piece finger location plate 13 is bowed like the troughs.

In order to activate a detecting terminal as shown in Figs. 1 and 2, a card holder inserts his plastic ID-card into the card reader 16. Evaluation logic starts a preliminary check of the ID-card, and, if the card is valid, activates a control lamp 29 which indicates that the card holder should locate his fingers on the finger location plate 13. The fingerprint verification process then starts.

The result of the verification process is then displayed on the display means 14 by phrases like "enter code on keyboard" or "rejected" or any other instructions to the card holder, such as instructions to place his other hand on the location plate 13 in case the fingerprint details are not detectable on one finger due to dust, grease or any other obstacles.

## Claims

1. Fingerprint detecting terminal comprising a fingerprint sensor with a contact surface (18) for a finger (19) under investigation and an ergonomically optimised finger location plate (13) comprising a primary finger trough (26) for controlling axial location, transverse motion and angular twist of said finger, whereby said contact surface (18) is arranged within said finger location plate (13) and wherein said finger trough (26) is designed ergonomically to suit the relaxed hand shape and position characterised in that said contact surface (18) comprises means such as guiding rails (24) arranged in parallel to each other for sideways location of said finger (19) with respect to said contact surface (18).

2. Fingerprint detecting terminal according to claim 1 wherein said primary finger trough (26) comprises a distal joint location ridge (21) for controlling axial location of said finger relative to contact surface (18).

3. Fingerprint detecting terminal according to claim 1 or 2, further comprising secondary finger troughs (25, 27) arranged adjacent to said primary finger trough (26) and designed to receive neighbouring fingers of said finger (19) under investigation.

4. Fingerprint detecting terminal according to claim 3, wherein said secondary finger troughs (25, 27) and said primary finger trough (26) are arranged at an angle (28) with respect to each other.

5. Fingerprint detecting terminal according to claim 4, wherein said angle (28) suits a relaxed hand position and is within the range of 2-5°.

6. Fingerprint detecting terminal according to any of claims 1 to 5 wherein said finger location plate (13) is designed as a housing cover of said terminal.

7. Fingerprint detecting terminal according to claim 6, further comprising a box-like housing (10) for an entire evaluation logic and a power supply, whereby said housing (10) is covered by said finger location plate (13), an indication display (14), and an input keyboard (15).

8. Fingerprint detecting terminal according to any of claims 1 to 7 wherein said finger location plate (13) and said housing (10) carrying said finger location plate (13) are designed to form guiding elements (17) of a card reader to receive a card like data carrier placed between said guiding elements (17).

9. Fingerprint detecting terminal according to any of claims 2 to 8 wherein said location ridge (21) has a wedge-like shape to fit the distal joint (21) of a human finger.

## Ansprüche

1. Fingerabdruck-Erfassungsterminal mit einem Fingerabdruck-Sensor mit einer Kontaktfläche (18) für einen zu untersuchenden Finger (19) und einer ergonomisch optimierten Fingeraufnahmeplatte (13) mit einer ersten, einen Finger aufnehmenden Ausnehmung (26) zur Steuerung der axialen Lage, der Querbewegung und dem Krümmungswinkel des Fingers, wobei die Kontaktfläche (18) innerhalb der Fingeraufnahmeplatte (13) angeordnet ist und wobei die den Finger aufnehmende Ausnehmung (26) ergonomisch zur Anpassung an die entspannte Hand nach Form und Lage ausgestaltet ist, **dadurch gekennzeichnet**, daß die Kontaktfläche (18) Mittel, insbesondere parallel angeordnete Führungselemente (24) zur seitlichen Führung des Fingers in bezug auf die Kontaktfläche (18) aufweist.

2. Fingerabdruck-Erfassungsterminal nach Anspruch 1, **dadurch gekennzeichnet**, daß die erste einen Finger aufnehmende Ausnehmung (26) eine Erhebung (21) für das Fingerkuppengelenk zur Steuerung der axialen Lage des Fingers relativ zur Kontaktfläche (18) aufweist.

3. Fingerabdruck-Erfassungsterminal nach einem der Ansprüche 1 oder 2, **gekennzeichnet** durch zusätzliche zweite, an die erste angrenzende Ausnehmungen (25, 27) zur Aufnahme von dem zu untersuchenden Finger (19) benachbarten Fingern.

4. Fingerabdruck-Erfassungsterminal nach Anspruch 3, **dadurch gekennzeichnet**, daß die ersten und zweiten Ausnehmungen (25, 26, 27) in einem Winkel zueinander angeordnet sind.

5. Fingerabdruck-Erfassungsterminal nach Anspruch 4, **dadurch gekennzeichnet**, daß der Winkel (28) der entspannten Handposition angepaßt ist und etwa 2-5° beträgt.

6. Fingerabdruck-Erfassungsterminal nach einem der Ansprüche 1 bis 5, **dadurch gekennzeichnet**, daß die Fingeraufnahmeplatte (13) als Abdeckung des Terminals ausgestattet ist.

7. Fingerabdruck-Erfassungsterminal nach Anspruch 6, **gekennzeichnet** durch ein behälterartiges Gehäuse (10) zur Aufnahme einer Auswertelogik und einer Stromversorgung, das von der Fingeraufnahmeplatte (13), einer Anzeigeeinrichtung (14) und einer Eingabetastatur (15) abgedeckt ist.

8. Fingerabdruck-Erfassungsterminal nach einem der Ansprüche 1 bis 7, **dadurch gekennzeichnet**, daß die Fingeraufnahmeplatte (13) und das die Fingeraufnahmeplatte (13) aufnehmende Gehäuse (10) Führungselemente (17) einer Kartenleseeinrichtung bilden, zwischen denen ein kartenförmiger Datenträger eingeführt werden kann.

9. Fingerabdruck-Erfassungsterminal nach einem der Ansprüche 2 bis 8, **dadurch gekennzeichnet**, daß die Erhebung (21) eine keilförmige, dem Fingerkuppengelenk des menschlichen Fingers angepaßte Form aufweist.

## Revendications

1. Terminal de détection d'empreintes digitales, comprenant un capteur d'empreintes digitales équipé d'une surface de contact (18) pour un doigt (19) en cours d'examen, et une plaque de positionnement de doigts optimisée au point de vue ergonomique (13), comprenant une cuvette de doigt principale (526) destinée à commander la position axiale, le mouvement transversal et la torsion angulaire du doigt, la surface de contact (18) étant placée à l'intérieur de la plaque de positionnement de doigts (13), et dans lequel la cuvette de doigt (26) est conçue de façon ergonomique pour s'adapter à la forme et à la position d'une main relâchée, caractérisé en ce que la surface de contact (18) comprend des moyens tels que des rails de guidage (24) disposés parallèlement l'un à l'autre pour le positionnement latéral du doigt (19) par rapport à la surface de contact (18).

2. Terminal de détection d'empreintes digitales, selon la revendication 1, dans lequel la cuvette de doigt principale (26) comprend une nervure de positionnement d'articulation distale (21) destinée à fixer la position axiale du doigt par rapport à la surface de contact (18).

3. Terminal de détection d'empreintes digitales, selon la revendication 1 ou 2, comprenant en outre des cuvettes de doigts secondaires (25, 27) disposées dans des positions adjacentes à la cuvette de doigt principale (26), et prévues pour recevoir des doigts voisins du doigt (19) qui est examiné.

4. Terminal de détection d'empreintes digitales, selon la revendication 3, dans lequel les cuvettes de doigts secondaires (25, 27) et la cuvette de doigt principale (26) sont orientées avec un certain angle (28) entre elles.

5. Terminal de détection d'empreintes digitales selon la revendication 4, dans lequel l'angle précité (28) est adapté à une position relâchée de la main, et il est compris dans la plage de 2 à 5°.

6. Terminal de détection d'empreintes digitales, selon l'une quelconque des revendications 1 à 5, dans lequel la plaque de positionnement de doigts (13) est conçue de façon à constituer un couvercle du coffret du terminal.

7. Terminal de détection d'empreintes digitales selon la revendication 6, comprenant en outre un coffret (10) en forme de boîte destiné à contenir l'ensemble de la logique d'évaluation et une alimentation, et ce coffret (10) est recouvert par la plaque de positionnement de doigts (13), un dispositif de visualisation d'indications (14) et un clavier d'entrée d'information (15).

8. Terminal de détection d'empreintes digitales selon l'une quelconque des revendications 1 à 7, dans lequel la plaque de positionnement de doigts (13) et le coffret (10) qui supporte la plaque de positionnement de doigts (13), sont conçus pour former des éléments de guidage (17) d'un lecteur de cartes pour recevoir un support de données se présentant sous la forme d'une carte qui est placé entre les éléments de guidage (17).

9. Terminal de détection d'empreintes digitales selon l'une quelconque des revendications 2 à 8, dans lequel la nervure de positionnement (21) a une section transversale triangulaire pour s'adapter à l'articulation distale (20) d'un doigt humain.

FIG 1

FIG 2

# FIG 3

a

b

c

d

e

f

g

# FIG 4

a

b

c

d

# FIG 5

# FIG 6